# EUROPEAN PATENT SPECIFICATION

(11) **EP 4 125 896 B1**
(45) Date of publication and mention of the grant of the patent: **07.05.2025**
(21) Application number: 21714685.1
(22) Date of filing: 24.03.2021
(51) Int. Cl.: A61K 31/4545, A61P 3/04, A61P 3/10, A61K 9/20

(54) **TREATMENT OF TYPE 2 DIABETES WITH 2-[(4-{6-[(4-CYANO-2-FLUOROBENZYL)OXY]PYRIDIN-2-YL} PIPERIDIN-1-YL)METHYL]-1-[(2S)-OXETAN-2-YLMETHYL]-1H-BENZIMIDAZOLE-6-CARBOXYLIC ACID OR A PHARMACEUTICALLY SALT THEREOF**
BEHANDLUNG VON TYP 2 DIABETES MIT 2-[(4{6-((4-CYANO-2-FLUORBENZYL)OXY)PYRIDIN-2-YL}PIPERIDIN-1-YL)METHYL!-1-[(2S)-OXETAN-2-YLMETHYL)-1H-BENZIMIDAZOL-6-CARBONSÄURE ODER EINEM PHARMAZEUTISCHEN SALZ DAVON
TRAITEMENT DU DIABÈTE DE TYPE 2 AVEC DE L'ACIDE 2-[(4-{6-[(4-CYANO-2-FLUOROBENZYL) OXY]PYRIDIN-2-YL}PIPÉRIDIN-1-YL)MÉTHYL]-1-[(2S)-OXÉTAN-2-YLMÉTHYL]-1H-BENZIMIDAZOLE-6-CARBOXYLIQUE OU UN SEL PHARMACEUTIQUEMENT ACCEPTABLE CORRESPONDANT

(30) Priority: 27.03.2020 US 202063000787 P; 16.12.2020 US 202063126113 P; 11.01.2021 US 202163135870 P
(43) Date of publication of application: 08.02.2023
(60) Divisional application: 24223164.5
(73) Proprietor: Pfizer Inc., New York, NY 10001-2192 (US)
(72) Inventor: LEE, Kai Teck, Sandwich Kent CT13 9NJ (GB); MANTHENA, Sweta, Groton, Connecticut 06340 (US); SAXENA, Aditi Rao, Cambridge, Massachusetts 02139 (US)
(74) Representative: Pfizer
(86) International application number: PCT/IB2021/052430
(87) International publication number: WO 2021/191812

(56) References cited:
- WO-A1-2013/117963
- US-B2- 10 208 019
- SAXENA ADITI R. ET AL: "Efficacy and Safety of Oral Small Molecule Glucagon-Like Peptide 1 Receptor Agonist Danuglipron for Glycemic Control Among Patients With Type 2 Diabetes : A Randomized Clinical Trial", JAMA NETWORK OPEN, vol. 6, no. 5, 1 May 2023 (2023-05-01), pages e2314493, XP093146487, ISSN: 2574-3805, Retrieved from the Internet <URL:https://pubmed.ncbi.nlm.nih.gov/37213102/> DOI: 10.1001/jamanetworkopen.2023.14493
- TIEN NGUYEN: "Drug structures displayed for the first time in Orlando", DRUG DISCOVERY, 5 April 2019 (2019-04-05), pages 1 - 7, XP055803652, Retrieved from the Internet <URL:https://cen.acs.org/pharmaceuticals/drug-discovery/Drug-structures-displayed-first-time-in-Orlando/97/web/2019/04> [retrieved on 20210511]
- ANONYMOUS: "History of Changes for Study: NCT03985293: A 16 Week Study to Evaluate the Efficacy and Safety of PF-06882961 in Adults With Type 2 Diabetes Mellitus", CLINICALTRIALS.GOV ARCHIVE, 16 January 2020 (2020-01-16), pages 1 - 8, XP055803635, Retrieved from the Internet <URL:https://clinicaltrials.gov/ct2/history/NCT03985293?V_5=View#StudyPageTop> [retrieved on 20210511]
- GRIFFITH DAVID A. ET AL: "A small-molecule oral agonist of the human glucagon-like peptide-1 receptor", BIORXIV, 30 September 2020 (2020-09-30), pages 1 - 33, XP055803533, Retrieved from the Internet <URL:https://www.biorxiv.org/content/10.1101/2020.09.29.319483v1.full.pdf> [retrieved on 20210511], DOI: 10.1101/2020.09.29.319483
- ANONYMOUS: "Analyst and Investor Call to Review Presentations of Hemophilia A Gene Therapy at WFH and Oral GLP-1R Agonist at ADA", 18 June 2020 (2020-06-18), pages 1 - 20, XP055803619, Retrieved from the Internet <URL:https://s21.q4cdn.com/317678438/files/doc_presentations/2020/06/Full-Slide-Deck_InvestorCall_FINAL_06172020.pdf> [retrieved on 20210511]
- ANONYMOUS: "Internal Medicine", 1 September 2020 (2020-09-01), pages 1 - 26, XP055803607, Retrieved from the Internet <URL:https://s21.q4cdn.com/317678438/files/doc_presentations/2020/09/Internal-Medicine_FINAL.pdf> [retrieved on 20210511]

## Description

### FIELD OF INVENTION

The invention provides a pharmaceutical composition for use in a method for treating type 2 diabetes mellitus, the method comprising administering to a human in need thereof a pharmaceutical composition twice daily in an oral dosage form, wherein the pharmaceutical composition contains 2-[(4-{6-[(4-cyano-2-fluorobenzyl)oxy]pyridin-2-yl}piperidin-1-yl)methyl]-1-[(2S)-oxetan-2-ylmethyl]-1H-benzimidazole-6-carboxylic acid, or a pharmaceutically salt thereof [such as its 2-amino-2-(hydroxymethyl)propane-1,3-diol salt, also known as its tris salt].

### BACKGROUND OF THE INVENTION

Diabetes mellitus is a major public health concern because of its increasing prevalence and associated health risks. The disease is characterized by high levels of blood glucose resulting from defects in insulin production, insulin action, or both. Two major forms of diabetes mellitus are recognized, Type 1 and Type 2. Type 1 diabetes mellitus (T1DM) develops when the body's immune system destroys pancreatic beta cells, the only cells in the body that make the hormone insulin that regulates blood glucose. To survive, people with Type 1 diabetes must have insulin administered by injection or a pump. Type 2 diabetes mellitus (referred to generally as T2DM) usually results from insulin resistance and insufficient production of insulin to maintain an acceptable glucose level.

Currently, various pharmacological approaches are available for treating hyperglycemia in T2DM (Hampp, C. et al. Use of Antidiabetic Drugs in the U.S., 2003-2012, Diabetes Care 2014, 37, 1367-1374). These may be grouped into six major classes, each acting through a different primary mechanism: insulin secretagogues, biguanides, alphaglucosidase inhibitors, thiazolidinediones (TZDs), insulin and sodium-glucose linked transporter cotransporter 2 (SGLT2) inhibitors. (A) Insulin secretogogues include sulphonylureas (e.g., glipizide, glimepiride, glyburide), meglitinides (e.g., nateglidine, repaglinide), dipeptidyl peptidase IV (DPP-IV) inhibitors (e.g., sitagliptin, vildagliptin, alogliptin, dutogliptin, linagliptin, saxogliptin), and glucagon-like peptide-1 receptor (GLP-1R) agonists (e.g., liraglutide, albiglutide, exenatide, lixisenatide, dulaglutide, semaglutide), and they act on the pancreatic beta-cells to enhance secretion of insulin. Sulphonyl-ureas and meglitinides have limited efficacy and tolerability, cause weight gain and often induce hypoglycemia. DPP-IV inhibitors have limited efficacy. Marketed GLP-1R agonists are peptides administered primarily by subcutaneous injection. Liraglutide is additionally approved for the treatment of obesity. (B) Biguanides (e.g., metformin) are thought to act primarily by decreasing hepatic glucose production. Biguanides often cause gastrointestinal disturbances and lactic acidosis, further limiting their use. (C) Inhibitors of alpha-glucosidase (e.g., acarbose) decrease intestinal glucose absorption. These agents often cause gastrointestinal disturbances and/or have limited efficacy. (D) Thiazolidinediones (e.g., pioglitazone, rosiglitazone) act on a specific receptor (peroxisome proliferator-activated receptor-gamma) in the liver, muscle and fat tissues. They regulate lipid metabolism subsequently enhancing the response of these tissues to the actions of insulin. Frequent use of these drugs may lead to weight gain and may induce edema and anemia. (E) Insulin is used in more severe cases, either alone or in combination with the above agents, and frequent use may also lead to weight gain and carries a risk of hypoglycemia. (F) sodium-glucose linked transporter cotransporter 2 (SGLT2) inhibitors (e.g., dapagliflozin, empagliflozin, canagliflozin, ertugliflozin) inhibit reabsorption of glucose in the kidneys and thereby lower glucose levels in the blood. This emerging class of drugs may be associated with ketoacidosis and urinary tract infections.

However, except for GLP-1R agonists and SGLT2 inhibitors, the drugs have limited efficacy and do not address the most important problems, the declining β-cell function and the associated obesity.

Accumulation of too much storage fat can impair movement, flexibility, and alter the appearance of the body. Both overweight and obesity can be associated to or cause health problems.

Obesity is a chronic disease that is highly prevalent in modern society and is associated with numerous medical problems including hypertension, hypercholesterolemia, and coronary heart disease. It is further highly correlated with T2DM and insulin resistance, the latter of which is generally accompanied by hyperinsulinemia or hyperglycemia, or both. In addition, T2DM is associated with a two-to-four-fold increased risk of coronary artery disease. Presently, the only treatment that treats obesity with high efficacy is bariatric surgery, but this treatment is invasive and costly. Pharmacological intervention is generally less efficacious and associated with side effects. There is therefore an obvious need for more efficacious pharmacological intervention with fewer side effects and convenient administration.

Although T2DM is most commonly associated with hyperglycemia and insulin resistance, other diseases, conditions, symptoms, and complications associated with T2DM include diabetic neuropathy, diabetic nephropathy, diabetic retinopathy, obesity, dyslipidemia, hypertension, hyperinsulinemia, and nonalcoholic fatty liver disease (NAFLD), cardiovascular disease, and increased risk for cancer.

NAFLD is the hepatic manifestation of metabolic syndrome, and is a spectrum of hepatic conditions encompassing steatosis, non-alcoholic steatohepatitis (NASH), fibrosis, cirrhosis and ultimately hepatocellular carcinoma. NAFLD and NASH are considered the primary fatty liver diseases as they account for the greatest proportion of individuals with elevated hepatic lipids. The severity of NAFLD/NASH is based on the presence of lipid, inflammatory cell infiltrate, hepatocyte ballooning, and the degree of fibrosis. Although not all individuals with steatosis progress to NASH, a substantial portion does.

GLP-1 is a 30 amino acid long incretin hormone secreted by the L-cells in the intestine in response to ingestion of food. GLP-1 has been shown to stimulate insulin secretion in a physiological and glucose-dependent manner, decrease glucagon secretion, inhibit gastric emptying, decrease appetite, and stimulate proliferation of beta-cells. In non-clinical experiments GLP-1 promotes continued beta-cell competence by stimulating transcription of genes important for glucose-dependent insulin secretion and by promoting beta-cell neogenesis (Meier, et al. Biodrugs. 2003; 17 (2): 93-102).

In a healthy individual, GLP-1 plays an important role regulating post-prandial blood glucose levels by stimulating glucose-dependent insulin secretion by the pancreas resulting in increased glucose absorption in the periphery. GLP-1 also suppresses glucagon secretion, leading to reduced hepatic glucose output. In addition, GLP-1 delays gastric emptying and slows small bowel motility delaying food absorption. In people with T2DM, the normal post-prandial rise in GLP-1 is absent or reduced (Vilsboll T, et al. Diabetes. 2001. 50; 609-613).

Holst (Physiol. Rev. 2007, 87, 1409) and Meier (Nat. Rev. Endocrinol. 2012, 8, 728) describe that GLP-1 receptor agonists, such as GLP-1, liraglutide and exendin-4, have 3 major pharmacological activities to improve glycemic control in patients with T2DM by reducing fasting and postprandial glucose (FPG and PPG): (i) increased glucose-dependent insulin secretion (improved first- and second-phase), (ii) glucagon suppressing activity under hyperglycemic conditions, (iii) delay of gastric emptying rate resulting in retarded absorption of meal-derived glucose.

There remains a need for a safe and efficacious treatment for cardiometabolic and associated diseases, such as T2DM, obesity, and overweight.

2-[(4-{6-[(4-Cyano-2-fluorobenzyl)oxy]pyridin-2-yl}piperidin-1-yl)methyl]-1-[(2S)-oxetan-2-ylmethyl]-1H-benzimidazole-6-carboxylic acid, or a pharmaceutically salt thereof [such as its 2-amino-2-(hydroxymethyl)propane-1,3-diol salt, also known as its tris salt or its tris(hydroxyethyl)methylamine salt] is a GLP-1R agonist described in U.S. Patent No.10,208,019 (see Example 4A-01 of the patent).

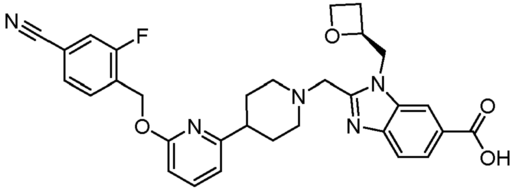

### 2-[(4-{6-[(4-Cyano-2-fluorobenzyl)oxy]pyridin-2-yl}piperidin-1-yl)methyl]-1-[(2S)-oxetan-2-ylmethyl]-1H-benzimidazole-6-carboxylic acid ("Compound 1").

Tris salt of 2-[(4-{6-[(4-Cyano-2-fluorobenzyl)oxy]pyridin-2-yl}piperidin-1-yl)methyl]-1-[(2S)-oxetan-2-ylmethyl]-1H-benzimidazole-6-carboxylic acid means a salt of Compound 1 made by using 1,3-dihydroxy-2-(hydroxymethyl)propan-2-amine. The tris is associated with the carboxylic acid moiety of Compound 1. Unless otherwise stated, when referencing the tris salt of Compound 1, the counterion and Compound 1 are in a stoichiometric ratio of about 1:1 (i.e. from 0.9:1.0 to 1.0:0.9, for example, from 0.95:1.00 to 1.00:0.95, or from 0.99:1.00 to 1.00 : 1.01). Another chemical name for tris salt of Compound 1 is 1,3-dihydroxy-2-(hydroxymethyl)propan-2-aminium 2-[(4-{6-[(4-cyano-2-fluorobenzyl)oxy]pyridin-2-yl}piperidin-1-yl)methyl]-1-[(2S)-oxetan-2-ylmethyl]-1H-benzimidazole-6-carboxylate, which can also be represented, for example, by one of the following structures. 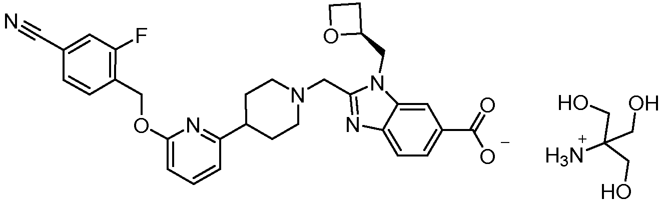 or

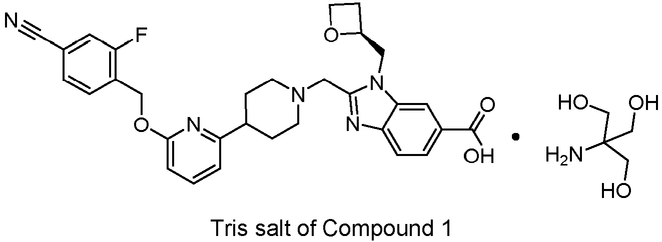

Information concerning study NCT03985293 may be found in the Clinical.Trials.gov archive. This was a 16 week study to evaluate the efficacy and safety of the compound PF-68882961 in adults with Type 2 Diabetes Mellitus.

### SUMMARY OF THE INVENTION

The present invention provides a pharmaceutical composition for use in a method for treating T2DM comprising administering to a human in need thereof the pharmaceutical composition, wherein:
the pharmaceutical composition is in an immediate release solid oral dosage form;
the pharmaceutical composition comprises 2-[(4-{6-[(4-cyano-2-fluorobenzyl)oxy]pyridin-2-yl}piperidin-1-yl)methyl]-1-[(2S)-oxetan-2-ylmethyl]-1H-benzimidazole-6-carboxylic acid (Compound 1) or a pharmaceutically acceptable salt thereof;
the pharmaceutical composition is administered twice daily; and
the pharmaceutical composition contains Compound 1 or a pharmaceutically acceptable salt thereof in an amount equivalent to 70 mg to 120 mg of Compound 1.

### BRIEF DESCRIPTION OF FIGURES

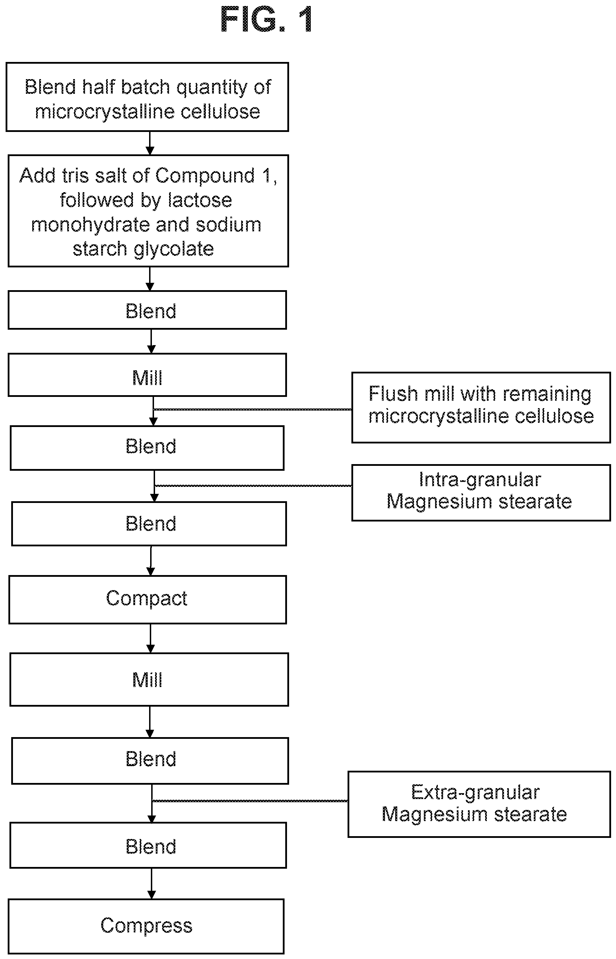

FIG. 1 shows a flow diagram of the preparation process of tris salt of Compound 1 immediate release tablets.
FIG. 2 shows a flow diagram of the preparation process of tris salt of Compound 1 (eqivalent to 50 mg of Compound 1) controlled release tablets.

### DETAILED DESCRIPTION OF THE INVENTION

The present invention provides a pharmaceutical composition for use in a method for treating T2DM comprising administering to a human in need thereof the pharmaceutical composition, wherein:
the pharmaceutical composition is in an immediate release solid oral dosage form;
the pharmaceutical composition comprises 2-[(4-{6-[(4-cyano-2-fluorobenzyl)oxy]pyridin-2-yl}piperidin-1-yl)methyl]-1-[(2S)-oxetan-2-ylmethyl]-1H-benzimidazole-6-carboxylic acid (Compound 1) or a pharmaceutically acceptable salt thereof;
the pharmaceutical composition is administered twice daily; and
the pharmaceutical composition contains Compound 1 or a pharmaceutically acceptable salt thereof in an amount equivalent to 70 mg to 120 mg of Compound 1.

The pharmaceutical composition is present in a solid oral dosage form, which includes, for example, tablets, capsules, caplets, sachets, powders, granules, or orally dispersible films.

As use herein, the term "immediate release" or its abbreviated term "IR" [for example, in "immediate release tablet"] corresponds to the definition provided in European Pharmacopeia 6.0, part 01/2008: 1502 as relating to "conventional-release dosage forms" or "immediate-release dosage forms" in the form of a tablet showing a release of the active substance, which is not deliberately modified by a special formulation design and/or manufacturing method, thereby being distinct from "modify-release", "prolong-release", "delayed-release" and "pulsatile-release" dosage forms as defined in European Pharmacopeia 6.0., part 01/2008: 1502. For example, more specifically, "immediate release" or "IR" means a release quantity of the active pharmacological ingredient of at least 70%, 75%, or 80% within a defined time, such as 60 minutes, 45 minutes, or 30 minutes, as determined according to the USP release method using apparatus 2 (paddle), for example, having a Q value (30 minutes) of at least 75 %.

In some embodiments, the pharmaceutical composition includes one or more tablets.

In some embodiments, the pharmaceutical composition contains Compound 1 or a pharmaceutically salt thereof in an amount equivalent to 120 mg of Compound 1.

In some embodiments, the Compound 1 or pharmaceutically salt thereof in the pharmaceutical composition is a pharmaceutical salt of Compound 1, for example, tris salt of Compound 1.

The pharmaceutical composition is administered twice daily. In some embodiments, the two daily administrations are separated by at least 4, 5, or 6 hours. In some embodiments, the two daily administrations are separated by at least 6, 7, or 8 hours. In some embodiments, the two daily administrations are separated by at least 8, 9, or 10 hours.

In some embodiments, the two daily administrations are separated by 4 to 16 hours, 8 to 16 hours, or 10 to 14 hours. In some further embodiments, the two daily administrations are separated by 11 to 13 hours, or about 12 hours.

The term "treating", as used herein, unless otherwise indicated, means reversing, alleviating, inhibiting the progress of, or preventing the disorder or condition to which such term applies, or one or more symptoms of such disorder or condition. The term "treatment", as used herein, unless otherwise indicated, refers to the act of treating as "treating" is defined herein. The term "treating" also includes adjuvant and neo-adjuvant treatment of a subject (e.g. a human).

In some embodiments, the method for treating T2DM includes improving glycemic control.

In some embodiments, the method for treating T2DM includes reducing the fasting plasma glucose level of the human, for example, to about 126 mg/dL or lower. In some embodiments, the method treating T2DM includes reducing glycated hemoglobin (HbA1c), for example, to about 7.0 % or less, about 6.5% or less, or about 5.7% or less. In some embodiments, the method treating T2DM includes reducing the mean daily glucose level to about 157 mg/dL or less. In some embodiments, the method for treating T2DM has low or no risk of hypoglycemia.

In some embodiments, the method further includes administering to the human an additional therapeutic agent.

In some embodiments, the method is an adjunct to a reduced-calorie diet and/or increased physical activity.

One immediate-release oral pharmaceutical composition comprises:
Compound 1 or a pharmaceutically salt thereof (e.g. tris salt of Compound 1);
a filler [e.g. microcrystalline cellulose, lactose (e.g. in the form of lactose monohydrate), or a combination thereof, for example, a combination of microcrystalline cellulose and lactose monohydrate in about 2:1 weight ratio];
a disintegrant (e.g. crospovidone, starch, pregelatinized starch, carboxymethylcellulose, hydroxypropylcellulose, sodium alginate, croscarmellose sodium, or sodium starch glycolate),
and a lubricant [e.g. metallic stearate (such as magnesium stearate or sodium stearyl fumarate)].

In some embodiments, the oral pharmaceutical composition comprises microcrystalline cellulose, lactose (e.g. in the form of lactose monohydrate), sodium starch glycolate, and magnesium stearate.

In some embodiments, the oral pharmaceutical composition comprises microcrystalline cellulose, lactose (e.g. in the form of lactose monohydrate), sodium starch glycolate, and sodium stearyl fumarate.

In some embodiment, the oral pharmaceutical composition for use in the invention comprises:
1.0% to 35.0% by weight of Compound 1 or a pharmaceutically acceptable salt thereof (e.g. tris salt of Compound 1);
60% to 95% by weight of filler [e.g. microcrystalline cellulose, lactose (e.g. in the form of lactose monohydrate), or a combination thereof, for example, a combination of microcrystalline cellulose and lactose monohydrate in about 2:1 weight ratio];
0.2% to 2.5% by weight lubricant [e.g. metallic stearate (such as magnesium stearate or sodium stearyl fumarate)]; and
1.0% to 5.0% by weight of disintegrant (e.g. crospovidone, starch, pregelatinized starch, carboxymethylcellulose, hydroxypropylcellulose, sodium starch glycolate or croscarmellose sodium).

In some embodiment, the oral pharmaceutical composition for use in the invention comprises:
1.0% to 35.0% by weight of Compound 1 or a pharmaceutically acceptable salt thereof (e.g. tris salt of Compound 1);
60% to 95% by weight of filler [e.g. microcrystalline cellulose, lactose (e.g. in the form of lactose monohydrate), or a combination thereof, for example, a combination of microcrystalline cellulose and lactose monohydrate in about 2:1 weight ratio];
0.2% to 2.5% by weight lubricant [e.g. metallic stearate (such as magnesium stearate or sodium stearyl fumarate)]; and
1.0% to 5.0% by weight of disintegrant (e.g. sodium starch glycolate or croscarmellose sodium).

In some embodiment, the oral pharmaceutical composition for use in the invention comprises:
10.0% to 35.0% by weight of Compound 1 or a pharmaceutically acceptable salt thereof (e.g. tris salt of Compound 1);
60% to 90% by weight of filler [e.g. microcrystalline cellulose, lactose (e.g. in the form of lactose monohydrate), or a combination thereof, for example, a combination of microcrystalline cellulose and lactose monohydrate in about 2:1 weight ratio];
0.2% to 2.5% by weight lubricant [e.g. metallic stearate (such as magnesium stearate or sodium stearyl fumarate)]; and
1.0% to 5.0% by weight of disintegrant (e.g. crospovidone, starch, pregelatinized starch, carboxymethylcellulose, hydroxypropylcellulose, sodium starch glycolate or croscarmellose sodium).

In some embodiment, the oral pharmaceutical composition for use in the invention comprises:
1.0% to 20.0% by weight of Compound 1 or a pharmaceutically acceptable salt thereof (e.g. tris salt of Compound 1);
70% to 95% by weight of filler [e.g. microcrystalline cellulose, lactose (e.g. in the form of lactose monohydrate), or a combination thereof, for example, a combination of microcrystalline cellulose and lactose monohydrate in about 2:1 weight ratio];
0.2% to 2.0% by weight lubricant [e.g. metallic stearate (such as magnesium stearate or sodium stearyl fumarate)]; and
1.0% to 5.0% by weight of disintegrant (e.g. sodium starch glycolate or croscarmellose sodium).

In some embodiment, the oral pharmaceutical composition for use in the invention comprises:
1.0% to 20.0% by weight of Compound 1 or a pharmaceutically acceptable salt thereof (e.g. tris salt of Compound 1);
70% to 95% by weight of filler [e.g. microcrystalline cellulose, lactose (e.g. in the form of lactose monohydrate), or a combination thereof];
0.5% to 1.5% by weight lubricant [e.g. metallic stearate (such as magnesium stearate or sodium stearyl fumarate)]; and
2.0% to 4.0% by weight of disintegrant (e.g. sodium starch glycolate or croscarmellose sodium).

In some embodiment, the oral pharmaceutical composition for use in the invention comprises:
7.0% to 18.0% by weight of Compound 1 or a pharmaceutically acceptable salt thereof (e.g. tris salt of Compound 1);
75% to 90% by weight of filler [e.g. microcrystalline cellulose, lactose (e.g. in the form of lactose monohydrate), or a combination thereof];
0.5% to 1.5% by weight lubricant [e.g. metallic stearate (such as magnesium stearate or sodium stearyl fumarate)]; and
2.5% to 3.5% by weight of disintegrant (e.g. sodium starch glycolate or croscarmellose sodium).

In some embodiment, the oral pharmaceutical composition for use in the invention comprises:
15.0% to 35.0% by weight of Compound 1 or a pharmaceutically acceptable salt thereof (e.g. 15.0% to 35.0% by weight of tris salt of Compound 1);
60.0% to 80.0% by weight of filler [e.g. microcrystalline cellulose, lactose (e.g. in the form of lactose monohydrate), or a combination thereof];
1.5% to 2.5% by weight lubricant [e.g. metallic stearate (such as magnesium stearate or sodium stearyl fumarate)]; and
2.5% to 3.5% by weight of disintegrant (e.g. sodium starch glycolate or croscarmellose sodium).

In some embodiments, the oral pharmaceutical composition comprises:
Compound 1 or a pharmaceutically salt thereof (e.g. tris salt of Compound 1);
a filler [e.g. microcrystalline cellulose, lactose (e.g. in the form of lactose monohydrate), or a combination thereof, for example, a combination of microcrystalline cellulose and lactose monohydrate in about 2:1 weight ratio];
a disintegrant (e.g. crospovidone, starch, pregelatinized starch, carboxymethylcellulose, hydroxypropylcellulose); and
a lubricant [e.g. metallic stearate (such as magnesium stearate or sodium stearyl fumarate)].

In some embodiments, the oral pharmaceutical composition comprises microcrystalline cellulose, lactose (e.g. in the form of lactose monohydrate), crospovidone, and magnesium stearate.

In some embodiment, the oral pharmaceutical composition for use in the invention comprises:
1.0% to 35.0% by weight of Compound 1 or a pharmaceutically acceptable salt thereof (e.g. tris salt of Compound 1);
60% to 95% by weight of filler [e.g. microcrystalline cellulose, lactose (e.g. in the form of lactose monohydrate), or a combination thereof, for example, a combination of microcrystalline cellulose and lactose monohydrate in about 2:1 weight ratio];
0.2% to 2.5% by weight lubricant [e.g. metallic stearate (such as magnesium stearate or sodium stearyl fumarate)]; and
1.0% to 5.0% by weight of disintegrant (e.g. crospovidone,).

In some embodiment, the oral pharmaceutical composition of the invention comprises:
10.0% to 35.0% by weight of Compound 1 or a pharmaceutically acceptable salt thereof (e.g. tris salt of Compound 1);
60% to 70% by weight of filler [e.g. microcrystalline cellulose, lactose (e.g. in the form of lactose monohydrate), or a combination thereof, for example, a combination of microcrystalline cellulose and lactose monohydrate in about 2:1 weight ratio];
0.2% to 2.5% by weight lubricant [e.g. metallic stearate (such as magnesium stearate or sodium stearyl fumarate)]; and
1.0% to 5.0% by weight of disintegrant (e.g. crospovidone).

In some embodiment, the oral pharmaceutical composition for use in the invention comprises:
23.0% to 35.0% by weight of Compound 1 or a pharmaceutically acceptable salt thereof (e.g. 29.0% to 32.0% by weight of tris salt of Compound 1);
60% to 70% by weight of filler [e.g. microcrystalline cellulose, lactose (e.g. in the form of lactose monohydrate), or a combination thereof, for example, a combination of microcrystalline cellulose and lactose monohydrate in about 2:1 weight ratio];
1.0% to 2.5% by weight lubricant [e.g. metallic stearate (such as magnesium stearate or sodium stearyl fumarate)]; and
2.0% to 4.0% by weight of disintegrant (e.g. crospovidone).

In some embodiment, the oral pharmaceutical composition for use in the invention comprises:
24.0% to 33.0% by weight of Compound 1 or a pharmaceutically acceptable salt thereof (e.g. 29.0% to 32.0% by weight of tris salt of Compound 1);
62.0% to 68.0% by weight of filler [e.g. microcrystalline cellulose, lactose (e.g. in the form of lactose monohydrate), or a combination thereof, for example, a combination of microcrystalline cellulose and lactose monohydrate in about 2:1 weight ratio];
1.5% to 2.5% by weight lubricant [e.g. metallic stearate (such as magnesium stearate or sodium stearyl fumarate)]; and
2.5% to 3.5% by weight of disintegrant (e.g. crospovidone).

The immediate-release oral dosage form is a solid oral dosage form, which includes, for example, tablets, capsules, caplets, sachets, powders, granules, orally dispersible films. In some embodiments, the immediate-release oral dosage form is a tablet form.

In some embodiments, the tris salt of Compound 1 is present in a crystalline form, for example, the one disclosed in U.S. Patent No.10,208,019 (see Example 4A-01 of the patent).

In some embodiments, the Compound 1 in the pharmaceutical composition is present in amorphous form of Compound 1 or in amorphous form of tris salt Compound 1. Amorphous form of Compound 1 or amorphous form of tris salt of Compound 1 can be prepared by, for example, lyophilization (freeze dry).

The term "about" generally means within 5%, preferably within 3%, and more preferably within 1% of a given value or range. Alternatively, the term "about" means within an acceptable standard error of the mean, when considered by one skilled in the art.

The term "tris" means 1,3-dihydroxy-2-(hydroxymethyl)propan-2-amine, also known as THAM, tromethamine, 2-amino-2-(hydroxymethyl)propane-1,3-diol, tris(hydroxymethyl)aminomethane.

Tris salt of Compound 1 means a salt of Compound 1 made using 1,3-dihydroxy-2-(hydroxymethyl)propan-2-amine. The tris is associated with the carboxylic acid moiety of Compound 1. Unless otherwise stated, when referencing the tris salt of Compound 1, the counterion and Compound 1 are in a stoichiometric ratio of about 1:1 (i.e. from 0.9:1.0 to 1.0:0.9, for example, from 0.95:1.00 to 1.00:0.95). Another chemical name for tris salt of Compound 1 is 1,3-dihydroxy-2-(hydroxymethyl)propan-2-aminium 2-[(4-{6-[(4-cyano-2-fluorobenzyl)oxy]pyridin-2-yl}piperidin-1-yl)methyl]-1-[(2S)-oxetan-2-ylmethyl]-1H-benzimidazole-6-carboxylate, which can also be represented, for example, by one of the following structures. 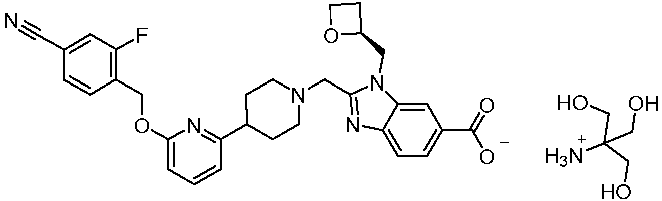 or

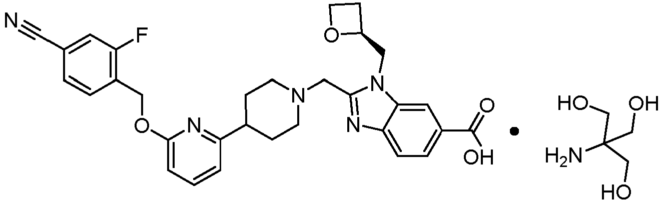

Those skilled in the art would readily understand that multiple nomenclatures can be used to name a same compound (including a same salt).

Pharmaceutically acceptable salts include acid addition and base salts.

Suitable acid addition salts are formed from acids which form non-toxic salts. Examples include the acetate, adipate, aspartate, benzoate, besylate, bicarbonate/carbonate, bisulfate/sulfate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate, 1,5-naphathalenedisulfonic acid and xinafoate salts.

Suitable base salts are formed from bases which form non-toxic salts. Examples include the aluminium, arginine, benzathine, calcium, choline, diethylamine, bis(2-hydroxyethyl)amine (diolamine), glycine, lysine, magnesium, meglumine, 2-aminoethanol (olamine), potassium, sodium, 2-Amino-2-(hydroxymethyl)propane-1,3-diol (tris or tromethamine) and zinc salts.

Hemisalts of acids and bases may also be formed, for example, hemisulfate and hemicalcium salts. For a review on suitable salts, see Handbook of Pharmaceutical Salts: Properties, Selection, and Use by Stahl and Wermuth (Wiley-VCH, 2002).

Pharmaceutically acceptable salts may be prepared by one or more of three methods:
(i) by reacting a compound with the desired acid or base;
(ii) by removing an acid- or base-labile protecting group from a suitable precursor of a compound or by ring-opening a suitable cyclic precursor, for example, a lactone or lactam, using the desired acid or base; or
(iii) by converting one salt of a compound to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

Compounds and pharmaceutically acceptable salts, may exist in unsolvated and solvated forms. The term 'solvate' is used herein to describe a molecular complex comprising a compound or its salt, and one or more pharmaceutically acceptable solvent molecules, for example, ethanol. The term 'hydrate' is employed when said solvent is water. For example, a hydrate crystalline form of tris salt of Compound 1 disclosed herein refers to a crystalline material/complex that includes both tris salt of Compond 1 and water (hydrate water) in the crystal lattice of the crystalline material/complex.

A currently accepted classification system for organic hydrates is one that defines isolated site, channel, or metal-ion coordinated hydrates - see Polymorphism in Pharmaceutical Solids by K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

When the solvent or water is tightly bound, the complex may have a well-defined stoichiometry independent of humidity. When, however, the solvent or water is weakly bound, as in channel solvates and hygroscopic compounds, the water/solvent content may be dependent on humidity and drying conditions. In such cases, non-stoichiometry will be the norm.

Compound 1 may be used in the form of a multi-component complex (other than a salts or solvate) wherein the drug and at least one other component are present in stoichiometric or non-stoichiometric amounts. Complexes of this type include clathrates (drug-host inclusion complexes) and co-crystals. The latter are typically defined as crystalline complexes of neutral molecular constituents which are bound together through non-covalent interactions, but could also be a complex of a neutral molecule with a salt. Co-crystals may be prepared by melt crystallisation, by recrystallisation from solvents, or by physically grinding the components together - see Chem Commun, 17, 1889-1896, by O. Almarsson and M. J. Zaworotko (2004). For a general review of multi-component complexes, see J Pharm Sci, 64 (8), 1269-1288, by Haleblian (August 1975).

Compound 1, or a pharmaceutically acceptable salt thereof, may exist in a continuum of solid states ranging from fully amorphous to fully crystalline. The term 'amorphous' refers to a state in which the material lacks long range order at the molecular level and, depending upon temperature, may exhibit the physical properties of a solid or a liquid. Typically such materials do not give distinctive X-ray diffraction patterns and, while exhibiting the properties of a solid, are more formally described as a liquid. Upon heating, a change from solid to liquid properties occurs which is characterised by a change of state, typically second order ('glass transition'). The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterised by a phase change, typically first order ('melting point').

A compound may also exist in a mesomorphic state (mesophase or liquid crystal) when subjected to suitable conditions. The mesomorphic state is intermediate between the true crystalline state and the true liquid state (either melt or solution). Mesomorphism arising as the result of a change in temperature is described as 'thermotropic' and that resulting from the addition of a second component, such as water or another solvent, is described as 'lyotropic'. Compounds that have the potential to form lyotropic mesophases are described as 'amphiphilic' and consist of molecules which possess an ionic (such as -COO⁻Na⁺, -COO⁻K⁺, or -SO₃⁻Na⁺) or non-ionic (such as -N⁻N⁺(CH₃)₃) polar head group. For more information, see Crystals and the Polarizing Microscope by N. H. Hartshorne and A. Stuart, 4th Edition (Edward Arnold, 1970).

Some compounds may exhibit polymorphism and/or one or more kinds of isomerism (e.g. optical, geometric or tautomeric isomerism). Compounds may also be isotopically labelled. Such variation is implicit to Compound 1 or its salt defined as they are by reference to their structural features and therefore within the scope of the invention.

Compounds containing one or more asymmetric carbon atoms can exist as two or more stereoisomers. Where a compound contains an alkenyl or alkenylene group, geometric cis/trans (or Z/E) isomers are possible. Where structural isomers are interconvertible via a low energy barrier, tautomeric isomerism ('tautomerism') can occur. This can take the form of proton tautomerism in compounds containing, for example, an imino, keto, or oxime group, or so-called valence tautomerism in compounds which contain an aromatic moiety. It follows that a single compound may exhibit more than one type of isomerism.

Certain pharmaceutically acceptable salts of Compound 1 may also contain a counterion which is optically active (e.g. d-lactate or I-lysine) or racemic (e.g. dl-tartrate or dl-arginine).

Cis/trans isomers may be separated by conventional techniques well known to those skilled in the art, for example, chromatography and fractional crystallisation.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC). Alternatively, a racemic precursor containing a chiral ester may be separated by enzymatic resolution (see, for example, Int J Mol Sci 29682-29716 by A. C. L. M. Carvaho et. al. (2015)). In the case where a compound contains an acidic or basic moiety, a salt may be formed with an optically pure base or acid such as 1-phenylethylamine or tartaric acid. The resulting diastereomeric mixture may be separated by fractional crystallization and one or both of the diastereomeric salts converted to the corresponding pure enantiomer(s) by means well known to a skilled person. Alternatively, the racemate (or a racemic precursor) may be covalently reacted with a suitable optically active compound, for example, an alcohol, amine or benzylic chloride. The resulting diastereomeric mixture may be separated by chromatography and/or fractional crystallization by means well known to a skilled person to give the separated diastereomers as single enantiomers with 2 or more chiral centers. Chiral compounds (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. Concentration of the eluate affords the enriched mixture. Chiral chromatography using sub-and supercritical fluids may be employed. Methods for chiral chromatography useful in some embodiments of the present invention are known in the art (see, for example, Smith, Roger M., Loughborough University, Loughborough, UK; Chromatographic Science Series (1998), 75 (SFC with Packed Columns), pp. 223-249 and references cited therein). In some relevant examples herein, columns were obtained from Chiral Technologies, Inc, West Chester, Pennsylvania, USA, a subsidiary of *Daicel*^{®} *Chemical Industries, Ltd., Tokyo, Japan.*

When any racemate crystallises, crystals of two different types are possible. The first type is the racemic compound (true racemate) referred to above wherein one homogeneous form of crystal is produced containing both enantiomers in equimolar amounts. The second type is the racemic mixture or conglomerate wherein two forms of crystal are produced in equimolar amounts each comprising a single enantiomer. While both of the crystal forms present in a racemic mixture have identical physical properties, they may have different physical properties compared to the true racemate. Racemic mixtures may be separated by conventional techniques known to those skilled in the art - see, for example, Stereochemistry of Organic Compounds by E. L. Eliel and S. H. Wilen (Wiley, 1994).

Although Compound 1 and its salts have been drawn herein in a single tautomeric form, all possible tautomeric forms are included within the scope of the invention.

The present invention includes all pharmaceutically acceptable isotopically-labeled Compound 1 or a salt thereof wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, nitrogen, such as ¹³N and ¹⁵N, and oxygen, such as ¹⁵O, ¹⁷O and ¹⁸O.

Certain isotopically-labelled Compound 1 or a salt thereof, for example those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection.

Substitution with heavier isotopes such as deuterium, i.e. ²H, may afford certain therapeutic advantages resulting from greater metabolic stability, for example, increased in vivo half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds can generally be prepared by conventional techniques known to those skilled in the art or by processes analogous to those described in the accompanying Examples and Preparations using an appropriate isotopically-labeled reagent in place of the non-labeled reagent previously employed.

Pharmaceutically acceptable solvates in accordance with the invention include those wherein the solvent of crystallization may be isotopically substituted, e.g. D₂O, d₆-acetone, d₆-DMSO.

### Administration and Dosing

Typically, Compound 1, or a pharmaceutically acceptable salt thereof, is administered in an amount effective to treat T2DM. Compound 1 can be administered per se, or alternatively, as a pharmaceutically acceptable salt. For administration and dosing purposes, the compound per se or pharmaceutically acceptable salt thereof will simply be referred to as the compounds for use in the invention.

The compounds for use in the invention are administered orally. Oral administration may involve swallowing, so that the compound enters the gastrointestinal tract, or buccal or sublingual administration may be employed by which the compound enters the bloodstream directly from the mouth.

The dosage regimen for the compounds for use in the invention and/or compositions containing said compounds is based on a variety of factors, including the type, age, weight, sex and medical condition of the patient; the severity of the condition; the route of administration; and the activity of the particular compound employed.

For oral administration, the compositions may be provided, for example, in the form of tablets containing the active ingredient for the symptomatic adjustment of the dosage to the patient.

Human subjects may be of either gender and at any stage of development.

### Pharmaceutical Compositions

Pharmaceutical compositions for use in the invention comprise a compound for use in the invention presented with a pharmaceutically acceptable carrier. Other pharmacologically active substances can also be present.

In some embodiments, pharmaceutically acceptable carriers include one or more components select from diluents/fillers, disintegrants, binders, wetting agents, and lubricants.

As used herein, the term "diluent or filler" refers to a substance that acts to dilute the active pharmacological agent to the desired dosage and/or that acts as a carrier for the active pharmacological agent. Examples of diluent or filler include mannitol, lactose (including e.g. lactose monohydrate), sucrose, maltodextrin, sorbitol, xylitol, powdered cellulose, microcrystalline cellulose, carboxymethylcellulose, carboxyethylcellulose, methylcellulose, ethylcellulose, hydroxyethylcellulose, methylhydroxyethylcellulose, starch, sodium starch glycolate, pregelatinized starch, a calcium phosphate, a metal carbonate, a metal oxide, and/or a metal aluminosilicate.

As used herein, the term "disintegrant" refers to a substance that encourages disintegration in water (or water-containing fluid *in vivo*) of a pharmaceutical composition/formulation of the invention. Examples of disintegrant include croscarmellose sodium, carmellose calcium, crospovidone, alginic acid, sodium alginate, potassium alginate, calcium alginate, an ion exchange resin, an effervescent system based on food acids and an alkaline carbonate component, clay, talc, starch, pregelatinized starch, sodium starch glycolate, cellulose floc, carboxymethylcellulose, hydroxypropylcellulose, calcium silicate, a metal carbonate, sodium bicarbonate, calcium citrate, and/or calcium phosphate.

As used herein, the term "binder" refers to a substance that increases the mechanical strength and/or compressibility of a pharmaceutical composition/formulation of the invention. Examples of binder include polyvinylpyrrolidone, copovidone, hydroxypropylcellulose, hydroxypropylmethylcellulose, crosslinked poly(acrylic acid), gum arabic, gum acacia, gum tragacanath, lecithin, casein, polyvinyl alcohol, gelatin, kaolin, cellulose, methylcellulose, hydroxymethylcellulose, carboxymethylcellulose, carboxymethylcellulose calcium, carboxymethylcellulose sodium, hydroxypropylcellulose, hydroxypropylmethylcellulose phthalate, hydroxyethylcellulose, methylhydroxyethylcellulose, silicified microcrystalline cellulose, starch, maltodextrin, dextrins, microcrystalline cellulose, and/or sorbitol.

As used herein, the term "wetting agent" refers to a substance that increases the water permeability of a pharmaceutical composition/formulation of the invention. In another aspect, the term, "wetting agent" refers to a substance that increases dissolution of the active pharmacological agent in water (or water containing fluid *in vivo*)*.* In yet another aspect, the term "wetting agent" refers to a substance that increases the bioavailability of the active pharmacological agent after administration of a pharmaceutical composition/formulation of the invention. Examples of wetting agent include metallic lauryl sulfate, polyethylene glycol, glycerides of fatty ester, polyoxyethylene-polyoxypropylene copolymer, polyoxyethylene-alkyl ether, metal alkyl sulfate, polyoxyethylene sorbitan fatty acid ester, polyoxyethylene castor oil derivative, sugar ester of fatty acid, polyglycolized glyceride, quaternary ammonium amine compound, lauroyl macrogol glycerides, caprylocaproyl macrogolglycerides, stearoyl macrogol glycerides, linoleoyl macrogol glycerides, oleoyl macrogol glycerides, polyethoxylated vegetable oil, polyethoxylated sterol, polyethoxylated cholesterol, polyethoxylated glycerol fatty acid ester, polyethoxylated fatty acid ester, sulfosuccinate, taurate, and/or docusate sodium.

As used herein, the term "lubricant" refers to a substance that aids in preventing sticking to the equipment of the pharmaceutical formulations/composition during processing and/or that improves powder flow of the composition/formulation during processing. Examples of lubricant include stearic acid, metallic stearate (e.g. magnesium stearate), sodium stearyl fumarate, fatty acid, fatty alcohol, fatty acid ester, glyceryl behenate, mineral oil, vegetable oil, paraffin, leucine, silica, silicic acid, talc, propylene glycol fatty acid ester, polyethylene glycol, polypropylene glycol, polyalkylene glycol, and/or sodium chloride.

In some embodiments, a composition for use in the invention comprises a filler [e.g. microcrystalline cellulose, lactose (e.g. in the form of lactose monohydrate), or a combination thereof], a disintegrant (e.g. croscarmellose sodium, sodium alginate, potassium alginate, or sodium starch glycolate), and a lubricant [e.g. metallic stearate (such as magnesium stearate)]. In some embodiments, a composition for use in the invention comprises microcrystalline cellulose, lactose (e.g. in the form of lactose monohydrate), sodium starch glycolate, and magnesium stearate.

Oral administration of a solid dose form may be, for example, presented in discrete units, such as hard or soft capsules, pills, cachets, lozenges, or tablets, each containing a predetermined amount of a pharmacological active ingredient (API, for example, Compound 1 or a pharmaceutical acceptable salt thereof such as tris salt of Compound 1). In one embodiment, the oral administration may be in tablet form. In one embodiment, the oral administration may be in capsule form. In one embodiment, the oral administration may be in a powder or granule form. In one embodiment, the oral dose form is sub-lingual, such as, for example, a lozenge. In such solid dosage forms, the compounds of the invention are ordinarily combined with one or more adjuvants. Such capsules or tablets contain an immediate release formulation.

Pharmaceutical compositions for use in the invention may be prepared by any of the well-known techniques of pharmacy, such as effective formulation and administration procedures. The above considerations in regard to effective formulations and administration procedures are well known in the art and are described in standard textbooks. Formulation of drugs is discussed in, for example, Hoover, John E., Remington's Pharmaceutical Sciences, Mack Publishing Co., Easton, Pennsylvania, 1975; Liberman et al., Eds., Pharmaceutical Dosage Forms, Marcel Decker, New York, N.Y., 1980; and Kibbe et al., Eds., Handbook of Pharmaceutical Excipients (3rd Ed.), American Pharmaceutical Association, Washington, 1999.

### Co-administration

The compositions for use in the invention can be used alone, or in combination with other therapeutic agents. The invention provides any of the uses, methods or compositions as defined herein wherein the compound of any embodiment herein, or pharmaceutically acceptable salt thereof, or pharmaceutically acceptable solvate of said compound or salt, is used in combination with one or more other therapeutic agent discussed herein. This would include a pharmaceutical composition for the treatment of a disease or condition for which an agonist of the GLP-1R is indicated, comprising a composition for use in the invention, as defined in any of the embodiments described herein, and one or more other therapeutic agent discussed herein.

The administration of two or more compounds "in combination" means that all of the compounds are administered closely enough in time that each may generate a biological effect in the same time frame. The presence of one agent may alter the biological effects of the other compound(s). The two or more compounds may be administered simultaneously, concurrently or sequentially. Additionally, simultaneous administration may be carried out by mixing the compounds prior to administration or by administering the compounds at the same point in time but as separate dosage forms at the same or different site of administration.

The phrases "concurrent administration," "co-administration," "simultaneous administration," and "administered simultaneously" mean that the compounds are administered in combination.

In another embodiment, the invention provides a pharmaceutical composition for use according to claim 1 in which Compound 1, or a pharmaceutically acceptable salt thereof, is administered in combination with one or more other pharmaceutical agents, wherein the one or more other pharmaceutical agents may be selected from the agents discussed herein.

In one embodiment, Compound 1, or a pharmaceutically acceptable salt thereof, is administered with an anti-diabetic agent including but not limited to a biguanide (e.g., metformin), a sulfonylurea (e.g., tolbutamide, glibenclamide, gliclazide, chlorpropamide, tolazamide, acetohexamide,glyclopyramide, glimepiride, or glipizide), a thiazolidinedione (e.g., pioglitazone, rosiglitazone, or lobeglitazone), a glitazar (e.g., saroglitazar, aleglitazar, muraglitazar or tesaglitazar), a meglitinide (e.g., nateglinide, repaglinide), a dipeptidyl peptidase 4 (DPP-4) inhibitor (e.g., sitagliptin, vildagliptin, saxagliptin, linagliptin, gemigliptin, anagliptin, teneligliptin, alogliptin, trelagliptin, dutogliptin, or omarigliptin), a glitazone (e.g., pioglitazone, rosiglitazone, balaglitazone, rivoglitazone, or lobeglitazone), a sodium-glucose linked transporter 2 (SGLT2) inhibitor (e.g., empagliflozin, canagliflozin, dapagliflozin, ipragliflozin, Ipragliflozin, tofogliflozin, sergliflozin etabonate, remogliflozin etabonate, or ertugliflozin), an SGLTL1 inhibitor, a GPR40 agonist (FFAR1/FFA1 agonist, e.g. fasiglifam), glucose-dependent insulinotropic peptide (GIP) and analogues thereof, an alpha glucosidase inhibitor (e.g. voglibose, acarbose, or miglitol), or an insulin or an insulin analogue, including the pharmaceutically acceptable salts of the specifically named agents and the pharmaceutically acceptable solvates of said agents and salts.

In another embodiment, Compound 1, or a pharmaceutically acceptable salt thereof, is administered with an anti-obesity agent including but not limited to peptide YY or an analogue thereof, a neuropeptide Y receptor type 2 (NPYR2) agonist, a NPYR1 or NPYR5 antagonist, a cannabinoid receptor type 1 (CB1R) antagonist, a lipase inhibitor (e.g., orlistat), a human proislet peptide (HIP), a melanocortin receptor 4 agonist (e.g., setmelanotide), a melanin concentrating hormone receptor 1 antagonist, a farnesoid X receptor (FXR) agonist (e.g. obeticholic acid), zonisamide, phentermine (alone or in combination with topiramate), a norepinephrine/dopamine reuptake inhibitor (e.g., buproprion), an opioid receptor antagonist (e.g., naltrexone), a combination of norepinephrine/dopamine reuptake inhibitor and opioid receptor antagonist (e.g., a combination of bupropion and naltrexone), a GDF-15 analog, sibutramine, a cholecystokinin agonist, amylin and analogues therof (e.g., pramlintide), leptin and analogues thereof (e.g., metroleptin), a serotonergic agent (e.g., lorcaserin), a methionine aminopeptidase 2 (MetAP2) inhibitor (e.g., beloranib or ZGN-1061), phendimetrazine, diethylpropion, benzphetamine, an SGLT2 inhibitor (e.g., empagliflozin, canagliflozin, dapagliflozin, ipragliflozin, Ipragliflozin, tofogliflozin, sergliflozin etabonate, remogliflozin etabonate, or ertugliflozin), an SGLTL1 inhibitor, a dual SGLT2/SGLT1 inhibitor, a fibroblast growth factor receptor (FGFR) modulator, an AMP-activated protein kinase (AMPK) activator, biotin, a MAS receptor modulator, or a glucagon receptor agonist (alone or in combination with another GLP-1R agonist, e.g., liraglutide, exenatide, dulaglutide, albiglutide, lixisenatide, or semaglutide), including the pharmaceutically acceptable salts of the specifically named agents and the pharmaceutically acceptable solvates of said agents and salts.

In another embodiment, Compound 1, or a pharmaceutically acceptable salt thereof, is administered in combination with one or more of the following: an agent to treat NASH including but not limited to PF-05221304, an FXR agonist (e.g., obeticholic acid), a PPAR α/δ agonist (e.g., elafibranor), a synthetic fatty acid-bile acid conjugate (e.g., aramchol), a caspase inhibitor (e.g., emricasan), an anti-lysyl oxidase homologue 2 (LOXL2) monoclonal antibody (e.g., simtuzumab), a galectin 3 inhibitor (e.g., GR-MD-02), a MAPK5 inhibitor (e.g., GS-4997), a dual antagonist of chemokine receptor 2 (CCR2) and CCR5 (e.g., cenicriviroc), a fibroblast growth factor 21 (FGF21) agonist (e.g., BMS-986036), a leukotriene D4 (LTD4) receptor antagonist (e.g., tipelukast), a niacin analogue (e.g., ARI 3037MO), an ASBT inhibitor (e.g., volixibat), an acetyl-CoA carboxylase (ACC) inhibitor (e.g., NDI 010976 or PF-05221304), a ketohexokinase (KHK) inhibitor, a diacylglyceryl acyltransferase 2 (DGAT2) inhibitor, a CB1 receptor antagonist, an anti-CB1R antibody, or an apoptosis signal-regulating kinase 1 (ASK1) inhibitor, including the pharmaceutically acceptable salts of the specifically named agents and the pharmaceutically acceptable solvates of said agents and salts.

Some specific compounds that can be used in combination with Compound 1, or a pharmaceutically acceptable salt thereof, for treating T2DM include:
4-(4-(1-Isopropyl-7-oxo-1,4,6,7-tetrahydrospiro[indazole-5,4'-piperidine]-1'-carbonyl)-6-methoxypyridin-2-yl)benzoic acid, which is an example of a selective ACC inhibitor and was prepared as the free acid in Example 9 of U.S. Patent No. 8,859,577, which is the U.S. national phase of International Application No. PCT/IB2011/054119. Crystal forms of 4-(4-(1-Isopropyl-7-oxo-1,4,6,7-tetrahydrospiro[indazole-5,4'-piperidine]-1'-carbonyl)-6-methoxypyridin-2-yl)benzoic acid, including an anhydrous mono-tris form (Form 1) and a trihydrate of the mono-tris salt (Form 2), are described in International PCT Application No. PCT/IB2018/058966;
(S)-2-(5-((3-Ethoxypyridin-2-yl)oxy)pyridin-3-yl)-N-(tetrahydrofuran-3-yl)pyrimidine-5-carboxamide, or a pharmaceutically acceptable salt thereof, and its crystalline solid forms (Form 1 and Form 2) is an example of a DGAT2 inhibitor described in Example 1 of U.S. Patent No. 10,071,992;
[(1R,5S,6R)-3-{2-[(2S)-2-methylazetidin-1-yl]-6-(trifluoromethyl)pyrimidin-4-yl}-3-azabicyclo[3.1.0]hex-6-yl]acetic acid, or a pharmaceutically acceptable salt thereof, (including a crystalline free acid form thereof) is an example of a ketohexokinase (KHK) inhibitor and is described in Example 4 of U.S. Patent No. 9,809,579; and
the FXR agonist Tropifexor or a pharmaceutically acceptable salt thereof is described in Example 1-1B of U.S. Patent No. 9,150,568.

### KITS

A kit comprising a pharmaceutical composition for use in the invention may be used in carrying out the invention. A kit may include, in addition to a pharmaceutical composition for use in the invention, diagnostic or therapeutic agents. A kit may also include instructions for use in a diagnostic or therapeutic method. In some embodiments, the kit includes a pharmaceutical composition for use in the invention and a diagnostic agent. In other embodiments, the kit includes a pharmaceutical composition and instructions for use in a therapeutic method.

In one embodiment, the kit contains one or more solid forms of a compound for use in the invention in quantities sufficient to carry out a method according to the invention. In another embodiment, the kit comprises one or more solid forms of a compound for use in the invention in quantities sufficient to carry out a method according to the invention and a container for the dosage.

The following examples illustrate the oral compositions/formulations and methods of the present invention.

### Example 1. Preparation of Immediate Release (IR) Tablets

Immediate release (IR), non-film-coated tablets of tris salt of Compound 1 in dosage strengths of 1 mg, 10 mg, 50 mg, and 100 mg were prepared [the dosage strength weight expressed in milligram is the weight equivalent to Compound 1].

The compositions of these tablets are shown in Tables 1-1 to 1-4.

**Table 1-1. Composition of tris salt of Compound 1 Tablet 1 mg**

| Name of Ingredients | Function | Unit Formula |
|---|---|---|
| tris salt of Compound 1 | Active Ingredient | 1.218 mg |
| Microcrystalline Cellulose | Filler | 63.188mg |
| Lactose Monohydrate | Filler | 31.594mg |
| Sodium Starch Glycolate | Disintegrant | 3.000 mg |
| Magnesium Stearate | Lubricant | 1.000 mg |
| Total Tablet Weight | | 100 mg |

**Table 1-2. Composition of tris salt of Compound 1 IR Tablet 10 mg**

| Name of Ingredients | Function | Unit Formula |
|---|---|---|
| tris salt of Compound 1 | Active Ingredient | 12.180 mg |
| Microcrystalline Cellulose | Filler | 55.880 mg |
| Lactose Monohydrate | Filler | 27.940 mg |
| Sodium Starch Glycolate | Disintegrant | 3.000 mg |
| Magnesium Stearate | Lubricant | 1.000 mg |
| Total Tablet Weight | | 100 mg |

**Table 1-3. Composition of tris salt of Compound 1 IR Tablet 50 mg**

| Name of Ingredients | Function | Unit Formula |
|---|---|---|
| tris salt of Compound 1 | Active Ingredient | 60.901 mg |
| Microcrystalline Cellulose | Filler | 471.399 mg |
| Lactose Monohydrate | Filler | 235.700 mg |
| Sodium Starch Glycolate | Disintegrant | 24.000 mg |
| Magnesium Stearate | Lubricant | 8.000 mg |
| Total Tablet Weight | | 800 mg |

**Table 1-4. Composition of tris salt of Compound 1 IR Tablet 100 mg**

| Name of Ingredients | Function | Unit Formula |
|---|---|---|
| tris salt of Compound 1 | Active Ingredient | 121.803 mg |
| Microcrystalline Cellulose | Filler | 430.798 mg |
| Lactose Monohydrate | Filler | 215.399 mg |
| Sodium Starch Glycolate | Disintegrant | 24.000 mg |
| Magnesium Stearate | Lubricant | 8.000 mg |
| Total Tablet Weight | | 800 mg |

### Process of preparing IR tablets

The following steps were carried out in preparing the IR tablets.
1. Blend approximately half of the microcrystalline cellulose.
2. Add tris salt of Compound 1 to the microcrystalline cellulose, followed by the lactose monohydrate and sodium starch glycolate, and mix.
3. Mill the blend and pass the remaining amount of microcrystalline cellulose through the mill. Blend the milled powder.
4. Add the intra-granular magnesium stearate and blend.
5. Compact and mill, then blend.
6. Add the extra-granular magnesium stearate and blend.
7. Compress using a suitable tablet press.

### Example 1B. Preparation of Additional Immediate Release (IR) Tablets

Additional immediate release (IR), non-film-coated tablets of tris salt of Compound 1 in dosage strengths of 100 mg were prepared [the dosage strength weight expressed in milligram is the weight equivalent to Compound 1]. The compositions of these tablets are shown in Tables 1-5, 1-6, and 1-7. These tablets were prepared using a process similar to that in Example 1 (the disintegrant used was sodium starch glycolate or cropovidone)

**Table 1-5. Composition of tris salt of Compound 1 Direct Compression (DC) IR Tablet 100mg**

| Name of Ingredients | Function | Unit Formula |
|---|---|---|
| tris salt of Compound 1 | Active Ingredient | 121.803 mg |
| Microcrystalline Cellulose | Filler | 172.132 mg |
| Lactose Monohydrate | Filler | 86.065 mg |
| Sodium Starch Glycolate | Disintegrant | 12.000 mg |
| Magnesium Stearate | Lubricant | 8.000 mg |
| Total Tablet Weight | | 400 mg |

**Table 1-6. Composition of tris salt of Compound 1 Direct Compression (DC) IR Tablet 100mg**

| Name of Ingredients | Function | Unit Formula |
|---|---|---|
| tris salt of Compound 1 | Active Ingredient | 121.803 mg |
| Microcrystalline Cellulose | Filler | 172.132 mg |
| Lactose Monohydrate | Filler | 86.065 mg |
| Crospovidone | Disintegrant | 12.000 mg |
| Magnesium Stearate | Lubricant | 8.000 mg |
| Total Tablet Weight | | 400 mg |

**Table 1-7. Composition of tris salt of Compound 1 Direct Compression (DC) IR Tablet 100mg**

| Name of Ingredients | Function | Unit Formula |
|---|---|---|
| tris salt of Compound 1 | Active Ingredient | 121.803 mg |
| Microcrystalline Cellulose | Filler | 172.132 mg |
| Lactose Monohydrate | Filler | 86.065 mg |
| Crospovidone | Disintegrant | 12.000 mg |
| Sodium Stearyl Fumarate | Lubricant | 8.000 mg |
| Total Tablet Weight | | 400 mg |

### Example 2. Preparation of Controlled Release (CR) Tablets (for reference only)

A film-coated controlled release (CR) tablet of tris salt of Compund 1 in dosage strength of 50 mg was prepared [the weight expressed in milligram is the weight equivalent to Compound 1].

The compositions of this CR tablet are shown in Table 2-1.

**Table 2-1. Composition of tris salt of Compound 1 CR Tablet (equivalent to 50 mg of Compound 1)**

| **Name of Ingredients** | **Function** | **Unit Formula (mg)** |
|---|---|---|
| Core Tablet | | |
| tris salt of Compound 1 | Active Ingredient | 60.901 |
| Polyethylene Oxide (200,000 molecular weight) | Entraining Polymer | 335.099 |
| Magnesium Stearate | Lubricant | 4.000 |
| Total Active Layer Weight | | 400.000 |
| Polyethylene Oxide (5,000,000 molecular weight) | Swelling Agent | 108.400 |
| Sodium Chloride | Osmogen | 60.000 |
| Microcrystalline Cellulose | Tableting Aid | 30.000 |
| Magnesium Stearate | Lubricant | 1.000 |
| FD&C Blue Aluminum Lake #2 | Colorant | 0.600 |
| Total Sweller Layer Weight | | 200.000 |
| **Total Bilayer Core Tablet Weight** | | 600.000 |
| Cellulose Acetate | Control Release | 42.500 |
| Polyethylene Glycol 3350 | Control Release | 7.500 |
| Acetone^{a} | Processing Aid | as required |
| Purified Water^{a} | Processing Aid | as required |
| Total Osmotic Coating Weight | | 50.000 |
| **Total Tablet Weight** | | **650.000 mg** |

| | | |
|---|---|---|
| ^{a} Removed during processing | | |

### Process of preparing CR tablets

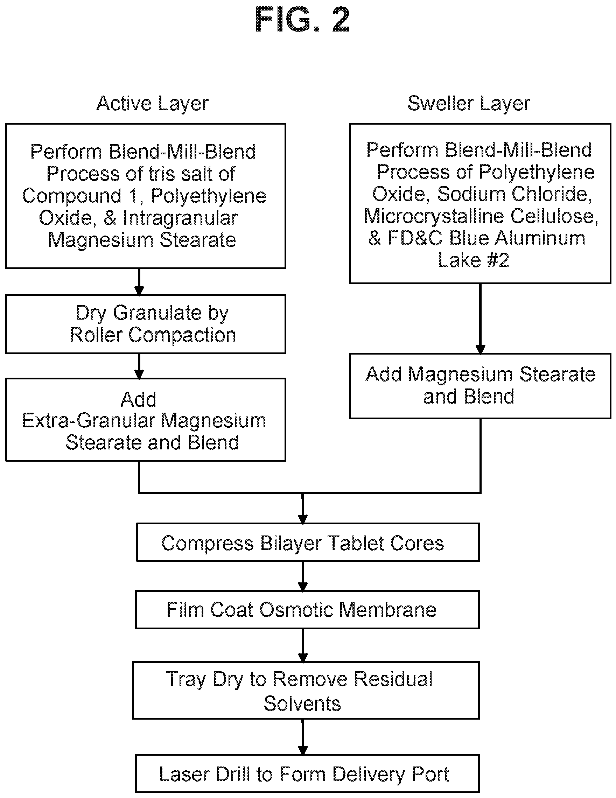

The following steps were carried out in preparing the CR tablets (See Fig. 2).

### Active Layer

1. Perform Blend-Mill-Blend Process of tris salt of Compound 1, Polyethylene Oxide, and a portion of the Magnesium Stearate using a 055R screen.
2. Dry granulate mixture from Step 1 by roller compaction.
3. Add the extra-granular Magnesium Stearate and blend the mixture to yield the active layer granulation.
   ***Sweller Layer***
4. Perform Blend-Mill-Blend Process of Polyethylene Oxide, Microcrystalline Cellulose, Sodium Chloride, and FD&C Blue Aluminum Lake #2 using a 055R screen.
5. Add Magnesium Stearate and blend the mixture to yield the sweller layer blend.
   ***Bilayer Tablet***
6. Compress the active layer and sweller layer into bilayer cores.
7. Film coat the bilayer cores using a suitable pan coater to produce the osmotic membrane.
8. Dry the tablets in a tray dryer to remove any residual amount of processing solvents.
9. Produce the delivery port on the active-layer face of the bilayer tablet using a suitable laser.

### Example 3. A clinical study (dose guiding study) of Compound 1 (in the form of its tris salt)

This study was a randomized, double-blind (sponsor-open), parallel, placebo-controlled, multiple oral dose-escalating study of Compound 1 (in the form of its tris salt) in participants with type 2 diabetes mellitus (T2DM) on a background of metformin monotherapy.

A total of 98 participants received oral doses of Compound 1 (in the form of its tris salt) or matching placebo for 28 days, in this study. A total of approximately 12 participants were enrolled in each cohort with a randomization ration of 3:1 (9 active and 3 placebo), and 8 cohorts were enrolled in the study. Participants were admitted to the clinical research unit (CRU) on or before Day -2 and were discharged following completion of all assessments on Day 30, at principal investigator discretion. For individual participants, the total duration of participation from the Screening visit to the on-site Follow-up visit was approximately 15 weeks. A Follow-up visit and a Follow-up contact (which have been conducted by phone call) occurred 35-42 days and 56-63 days following the first dose of investigational product on Day 1, respectively. Participants who discontinued prior to completion of the study might have been replaced, at the discretion of the principal investigator and Sponsor. 92 participants completed the inpatient study.

The planned titration schemes and dosing paradigms for all Cohorts are listed in Table 2-1.
QD: once daily
BID: twice daily

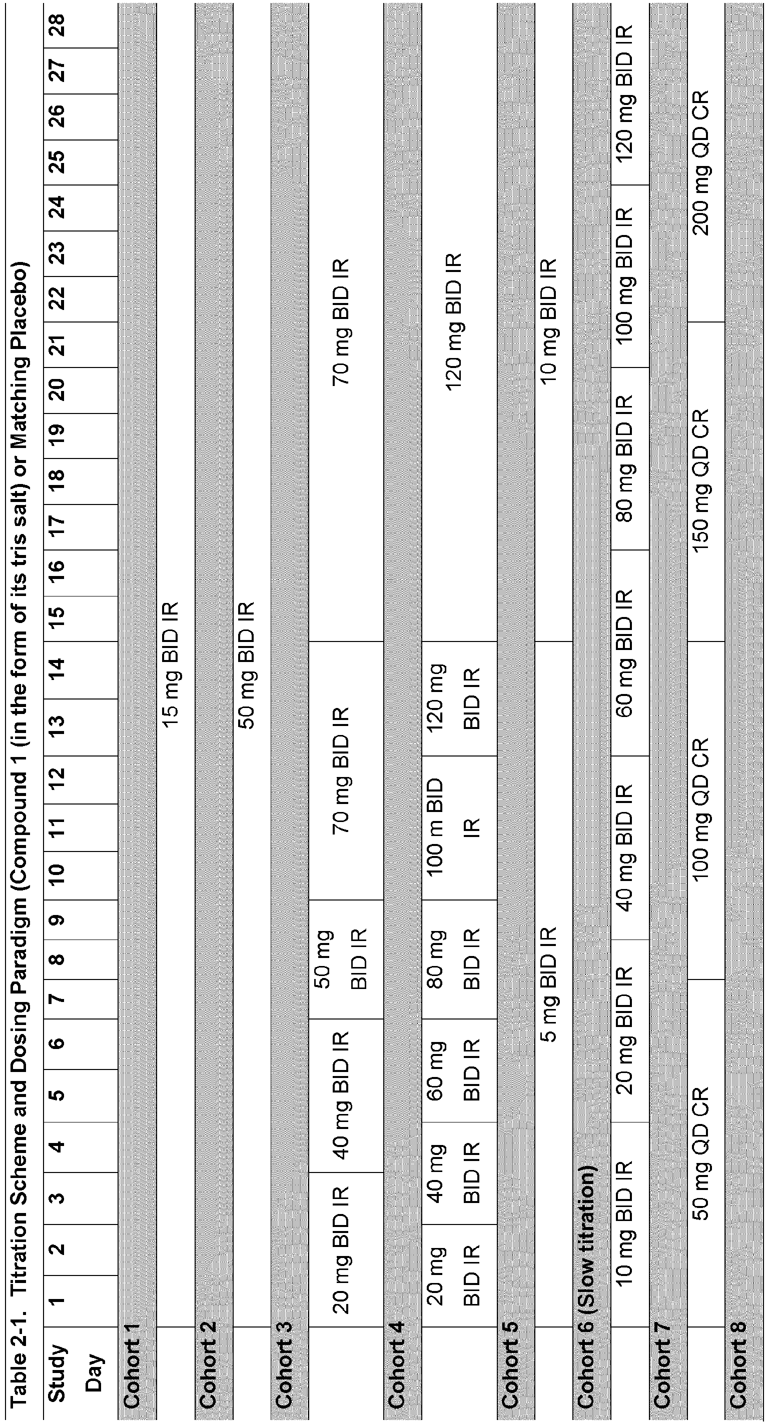

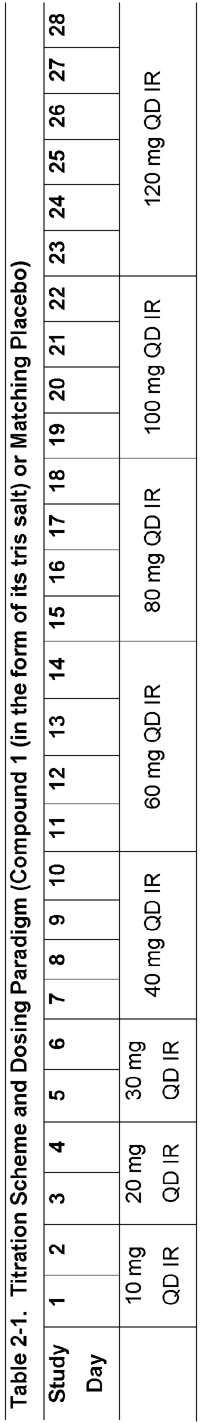

**Diagnosis and Main Criteria for Inclusion:** The population for this study was female participants of non-childbearing potential and/or male participants with T2DM, with an HbA1c ≥7.0% and ≤10.5%, who were taking metformin as their only anti-hyperglycemic treatment, and who were between 18 and 70 years with T2DM at the time of consent and Screening, with a body mass index (BMI) of 24.5 to 45.4 kg/m² and a total body weight >50 kg (110 lb). Metformin dose was required to be ≥500 mg per day and was required to be stable, for at least 2 months prior to the Screening visit and was administered in the CRU according to patient's baseline dosing regimen.

**Study Treatment:** Compound 1 (in the form of its tris salt) was supplied as 1 mg, 10 mg, 50 mg and 100 mg immediate release (IR) tablets or 50 mg controlled release (CR) tablets (see Examples 1 and 2) for oral administration. Matching placebo tablets (2:1, Microcrystalline Cellulose : Lactose) were also provided.

### Results

**Subject Disposition and Demography:** A total of 98 participants were randomized and assigned to receive study treatment, and 92 participants completed the study. Of the 6 participants who discontinued from the study, 2 participants discontinued due to treatment-related treatment-emergent AEs (TEAEs): 1 participant in Compound 1 (in the form of its tris salt) 15 mg twice daily (BID) group discontinued from the study due to a moderate TEAE of headache and 1 participant in the Compound 1 (in the form of its tris salt) 50 mg BID group discontinued from the study due to moderate TEAEs of decreased appetite, nausea, vomiting and a mild TEAE of fatigue. In addition, 1 participant in the Compound 1 (in the form of its tris salt) 50 mg BID group discontinued due to withdrawal by participant, and 3 participants from the Compound 1 (in the form of its tris salt) 200 mg once daily (QD) CR group discontinued due to other reasons. All discontinuations from the study occurred in the treatment phase. Demographic characteristics and physical measurements were generally comparable across dosing groups. The 98 randomized participants consisted of 51 males (52%) and 47 females (48%). The majority of the participants were White (70, 71.4%). Sixty-one (61, 62.2%) participants were Hispanic or Latino. The mean age of all participants was 57.4 years (range: 37 to 70 years). The mean weight was 92.4 kg (range: 50.2 to 138.9 kg). The mean BMI was 32.9 kg/m² (range: 25.0 to 43.0 kg/m²). The mean duration of T2DM for all participants was 9.5 years (range: 0.3 to 29.1 years), and baseline mean HbA1c for the study population was 8.3% (range: 8.01 to 8.61%).

### Safety Results:

### (A) Adverse Events

A total of 319 all causalities TEAEs were reported by 83 participants (84.7%) in the study, of which 262 (262/319: 82.1%) TEAEs were considered treatment-related. Two (2) participants experienced 2 severe TEAEs during the study, 1 of which occurred in the dosing period and was considered treatment-related, the other of which occurred during the follow-up period (also an SAE) and was not considered treatment-related. There were no deaths. One (1) SAE occurred in the study reporting period (not treatment-related and as previously mentioned). Another SAE, also not treatment-related, was reported in the same participant outside of the study reporting period and was not recorded in the clinical database A total of 2 participants discontinued from study due to TEAEs, and 2 participants discontinued from study drug due to TEAEs but continued in the study. Nine (9) participants had dose reduction or temporary discontinuation due to TEAEs: 1 in the placebo group, 4 in the 50 mg BID group, 1 in the 120 mg BID group, 2 in the 120 mg BID Slow titration group and 1 in the 200 mg QD CR group.

The Compound 1 (in the form of its tris salt) 10 mg BID and 15 mg BID groups had the lowest number of all causality TEAEs with 8 and 16 events, respectively. The 120 mg BID slow titration group had the largest number of all causality TEAEs with 50 events. One (1) SAE (not treatment-related) and 2 severe TEAEs (1 was treatment-related and 1 was not) were reported in the Compound 1 (in the form of its tris salt) 120mg BID slow titration group. The placebo, 50 mg BID, 70 mg BID, 120 mg BID, 120 mg QD and 200 mg QD CR groups reported 44, 45, 31, 43, 35 and 47 TEAEs, respectively.

The most frequently reported all causalities TEAEs by system organ class (SOC) were Gastrointestinal Disorders (reported in 73.5% of all participants) and Nervous System Disorders (33.7%). The most commonly reported al causalities TEAEs by preferred term (PT) were nausea (49.0%), dyspepsia (32.7%), vomiting (26.5%), diarrhea (24.5%), headache (23.5%) and constipation (20.4%). The incidence of all causalities TEAEs in Gastrointestinal Disorders category in each dose groups were higher than the incidence in the placebo group (52.0%) with an exception of the 10 mg BID group (33.3%).

The majority of all-causalities TEAEs (294 out of 319) were mild in severity. Twenty-three (23) TEAEs were moderate, of which 18 TEAEs were considered treatment-related. Two (2) severe TEAEs were reported in the 120 mg BID slow titration group, of which 1 was considered treatment-related.

One (1) participant in the 120 mg BID group experienced mild hypoglycemia after missing a meal or snack, which was self-limited and considered treatment-related.

### (B) Clinical Laboratory Evaluation

The most common laboratory abnormality, without regard to baseline abnormality, was hemoglobin A1C (%) >1.3 × upper limit of normal (ULN) (45, 48.9%), which was expected due to the eligibility criteria for the study. Other common laboratory abnormalities included activated partial thromboplastin time >1.1 × ULN (40, 40.8%), and urine glucose ≥1 mg/dL (36, 36.7%), which did not appear to be imbalanced relative to placebo.

### (C) Vital Signs

The most frequently reported post-baseline vital signs that met the pre-specified criteria for categorical analysis were supine systolic blood pressure (BP) decreased ≥30 mm Hg in 39 participants, and supine diastolic BP decreased ≥20 mm Hg in 24 participants across all treatment groups. None of the participants met the pre-specified criteria for pulse rate with values of <40 bpm and >120 bpm. There were no apparent dose-related increases in the frequency of vital sign abnormalities by categorical analysis.

Pulse rate increased with Compound 1 (in the form of its tris salt) 961 treatment compared to placebo at Days 1, 14, 21 and 28, with generally greater increases observed on Day 28 compared with Day 1. Across the dosing interval from Day 1 to Day 28, mean time-matched double differences in pulse rate ranged from -6.4 to 11.8 bpm across doses of Compound 1 (in the form of its tris salt) compared to a range of -6.4 to -0.8 bpm in placebo. While these increases in pulse rate were observed, no AEs were reported related to pulse rate and there were no occurrences of pulse rate >120 bpm.

### (D) ECG

A total of 5 participants reported post-baseline ECG (electrocardiogram) data meeting pre-specified criteria of aggregate QTcF (QT interval corrected for heart rate based on the Fridericia's correction formula) interval >450 and ≤480 or change from baseline >30 and ≤60, 2 of which occurred in the placebo group and 3 of which occurred in Compound 1 (in the form of its tris salt) planned treatment groups. These occurrences were self-limited and did not require intervention. No data met criteria of PR interval ≥300 msec or QRS duration ≥140 msec, and there were no apparent dose-related increases in the frequency of ECG abnormalities by categorical analysis. There were no ECG abnormalities in optional Compound 1 (in the form of its tris salt) groups (Compound 1 (in the form of its tris salt) 120 mg BID slow titration, 120 mg QD and 200 mg QD CR groups). No ECG abnormalities were reported as AEs and none of the ECG findings were reported as clinically significant by the investigator.

Ascending, multiple, oral doses of Compound 1 (in the form of its tris salt) were generally safe and well-tolerated in adult participants with T2DM. The most commonly reported all causalities TEAEs were nausea, dyspepsia, vomiting, diarrhea, headache and constipation. There were no clinically significant adverse trends in safety laboratory tests, vital signs or ECG parameters with increasing Compound 1 (in the form of its tris salt) dose.

### Pharmacokinetic Evaluations

Compound 1 (in the form of its tris salt) plasma pharmacokinetic parameters area under the plasma concentration time profile from time 0 to time 24 hours (AUC₂₄), maximum observed concentration over a 24-hour interval (Cₘₐₓ), and time for Cₘₐₓ (Tₘₐₓ) were calculated following Day 1, and multiple dose administration, were calculated for each participant and treatment, using noncompartmental analysis of concentration-time data.

| | |
|---|---|
| Cₘₐₓ | maximum observed concentration over a 24-hour interval |
| Cmax1 | maximum plasma concentration during the dosing interval τ1 =0 to 10 hours |
| Tₘₐₓ | time for Cₘₐₓ |
| Tₘₐₓ₁ | time for Cₘₐₓ₁ |
| t½ | terminal half life |
| HbA1c | glycated hemoglobin |
| AUC | area under the curve |
| AUC24 | area under the plasma concentration time profile from time 0 to time 24 hours |
| BMI | body mass index Body Mass Index (kg/m²) was defined as weight (kg) / [height (cm)×0.01]² |

Following a single oral dose of Compound 1 (in the form of its tris salt) on Day 1, absorption for the IR formulation for the QD and BID regimens occurred in a median Tₘₐₓ and Tₘₐₓ₁ of 2.0 to 4.0 hours post dose.

Day 1 exposure based on dose normalized mean AUC₂₄ values appeared to be approximately dose proportional across the BID and QD treatments for the IR formulation. On Day 28, AUC₂₄ and Cₘₐₓ increased in an approximately dose-proportional manner for cohorts administered BID IR formulation treatments.

Mean Cₘₐₓ and Cₘₐₓ₁ values on Day 28 were reached in a median Tₘₐₓ and Tₘₐₓ₁ of 3 to 10 hours post dose for the BID and QD IR treatments, while absorption was slower for the CR formulation treatment (Cohort 7), with a median Tmax of 14 hours post dose. Mean t½ values across all treatments ranged between 4.7 to 8.1 hours, and no apparent trends were observed across various treatments, regimens, or doses administered. The CR formulation exhibited approximately a 50% lower exposure (AUC₂₄ and Cₘₐₓ) on Day 14 compared to that observed for the IR formulations, however on Day 28 the exposure was similar to that observed for the IR formulation treatments. Urinary recovery of Compound 1 was low, with <0.1% of the dose recovered unchanged in urine on Day 28.

### FPG, MDG, HbA1C, and Body Weight Measurement

Measurements of fasting plasma glucose (FPG), 24-hour mean daily glucose (MDG), HbA1C, and body weight at baseline (at Day -1) and at Day 28 (shown as change from baselin) are shown in Tables 2-2 to 2-5.

Baseline was defined as the measurement (MDG, FPG, HbA1c, or body weight) on Day -1. Randomized treatment represented the target dose at Day 28.

Baseline was restricted to subjects who had a non-missing value for the change from baseline to the time point of interest

**Table 2-2. Summary of Baseline and Change from Baseline (CFB) for Mean Daily Glucose (mg/dL) by Randomized Treatment.**

| **Study Day** | **Randomized Treatment** | **N** | **Baseline (SD)** | **Mean CFB (SD)** |
|---|---|---|---|---|
| Day 28 | Placebo | 25 | 183.9 (38.30) | -20.5 (27.20) |
| | Compound 1 (in the form of its tris salt) 10mg BID | 9 | 189.9 (20.64) | -53.3 (21.80) |
| | Compound 1 (in the form of its tris salt) 15mg BID | 8 | 225.4 (38.78) | -85.9 (31.62) |
| | Compound 1 (in the form of its tris salt) 50mg BID | 8 | 190.2 (29.39) | -48.7 (36.29) |
| | Compound 1 (in the form of its tris salt) 70mg BID | 9 | 218.5 (45.89) | -101.6 (44.26) |
| | Compound 1 (in the form of its tris salt) 120mg BID | 9 | 219.0 (32.42) | -105.9 (33.64) |
| | Compound 1 (in the form of its tris salt) 120mg BID Slow Titration | 9 | 179.9 (36.69) | -68.6 (34.23) |
| | Compound 1 (in the form of its tris salt) 120mg QD | 8 | 188.8 (24.85) | -70.9 (23.65) |
| | Compound 1 (in the form of its tris salt) 200mg QD Controlled Release | 7 | 225.2 (44.80) | -102.8 (42.56) |

Observed data presented. Mean Daily Glucose (MDG) was defined as AUC₂₄/24.

**Table 2-3. Summary of Baseline and Change from Baseline (CFB) for Fasting Plasma Glucose (mg/dL) by Randomized Treatment**

| **Study Day** | **Randomized Treatment** | **N** | **Baseline (SD)** | **Mean CFB (SD)** |
|---|---|---|---|---|
| Day 28 | Placebo | 25 | 167.6 (32.5) | -24.8 (33.4) |
| | Compound 1 (in the form of its tris salt) 10mg BID | 9 | 158.3 (23.3) | -34.3 (37.7) |
| | Compound 1 (in the form of its tris salt) 15mg BID | 8 | 198.4 (33.1) | -66.6 (27.2) |
| | Compound 1 (in the form of its tris salt) 50mg BID | 8 | 168.1 (34.9) | -34.5 (47.0) |
| | Compound 1 (in the form of its tris salt) 70mg BID | 9 | 186.3 (32.0) | -80.6 (37.2) |
| | Compound 1 (in the form of its tris salt) 120mg BID | 9 | 196.9 (26.1) | -89.7 (30.0) |
| | Compound 1 (in the form of its tris salt) 120mg BID Slow Titration | 9 | 176.3 (44.0) | -75.1 (43.2) |
| | Compound 1 (in the form of its tris salt) 120mg QD | 8 | 179.9 (28.5) | -48.3 (42.8) |
| | Compound 1 (in the form of its tris salt) 200mg QD Controlled Release | 7 | 195.3 (37.5) | -77.3 (44.7) |

**Table 2-4. Baseline and Change from Baseline (CFB) for HbA1c (%) by Randomized Treatment.**

| **Study Day** | **Randomized Treatment** | **N** | **Baseline (SD)** | **Mean CFB (SD)** |
|---|---|---|---|---|
| Day 28 | Placebo | 25 | 8.0 (0.8) | -0.4 (0.4) |
| | Compound 1 (in the form of its tris salt) 10mg BID | 9 | 8.2 (0.6) | -0.8 (0.5) |
| | Compound 1 (in the form of its tris salt) 15mg BID | 8 | 8.6 (0.6) | -0.9 (0.3) |
| | Compound 1 (in the form of its tris salt) 50mg BID | 8 | 8.3 (0.9) | -1.2 (0.6) |
| | Compound 1 (in the form of its tris salt) 70mg BID | 9 | 8.3 (0.6) | -1.2 (0.3) |
| | Compound 1 (in the form of its tris salt) 120mg BID | 9 | 8.5 (1.0) | -1.2 (0.6) |
| | Compound 1 (in the form of its tris salt) 120mg BID Slow Titration | 9 | 8.2 (0.7) | -1.2 (0.5) |
| | Compound 1 (in the form of its tris salt) 120mg QD | 8 | 8.2 (1.0) | -1.0 (0.6) |
| | Compound 1 (in the form of its tris salt) 200mg QD Controlled Release | 7 | 8.6 (0.9) | -1.1 (0.4) |

**Table 2-5. Summary of Baseline and Change from Baseline (CFB) for Body Weight (Kg) by Randomized Treatment**

| **Study Day** | **Randomized Treatment** | **N** | **Baseline (SD)** | **Mean CFB (SD)** |
|---|---|---|---|---|
| Day 28 | Placebo | 25 | 94.26 (17.74) | -1.9 (2.3) |
| | Compound 1 (in the form of its tris salt) 10mg BID | 9 | 99.90 (12.44) | -2.8 (1.8) |
| | Compound 1 (in the form of its tris salt) 15mg BID | 8 | 93.60 (22.47) | -2.4 (2.0) |
| | Compound 1 (in the form of its tris salt) 50mg BID | 8 | 88.24 (13.91) | -2.4 (2.3) |
| | Compound 1 (in the form of its tris salt) 70mg BID | 9 | 86.08 (18.64) | -4.0 (2.0) |
| | Compound 1 (in the form of its tris salt) 120mg BID | 9 | 101.34 (17.51) | -7.9 (3.5) |
| | Compound 1 (in the form of its tris salt) 120mg BID Slow Titration | 9 | 88.01 (19.89) | -5.5 (2.7) |
| | Compound 1 (in the form of its tris salt) 120mg QD | 8 | 84.88 (15.50) | -4.3 (3.3) |
| | Compound 1 (in the form of its tris salt) 200mg QD Controlled Release | 7 | 92.29 (18.01) | -3.1 (2.1) |

As shown in Tables 2-2 to 2-4, Compound 1 (in the form of its tris salt) showed robust reductions in FPG, MDG, HbA1c, and body weight at Day 28, with increasing efficacy at higher doses of 70 mg to 120 mg twice daily.

## Claims

1. A pharmaceutical composition for use in a method for treating T2DM comprising administering to a human in need thereof the pharmaceutical composition, wherein:
the pharmaceutical composition is in an immediate release solid oral dosage form;
the pharmaceutical composition comprises 2-[(4-{6-[(4-cyano-2-fluorobenzyl)oxy]pyridin-2-yl}piperidin-1-yl)methyl]-1-[(2S)-oxetan-2-ylmethyl]-1H-benzimidazole-6-carboxylic acid (Compound 1) or a pharmaceutically acceptable salt thereof;
the pharmaceutical composition is administered twice daily; and
the pharmaceutical composition contains Compound 1 or a pharmaceutically acceptable salt thereof in an amount equivalent to 70 mg to 120 mg of Compound 1.

2. A pharmaceutical composition for use in a method for treating T2DM as claimed in claim 1, wherein the pharmaceutical composition is in an immediate-release tablet dosage form.

3. A pharmaceutical composition for use in a method for treating T2DM as claimed in any one of claims 1 to 2, wherein the Compound 1 or pharmaceutically salt thereof in the pharmaceutical composition is tris salt of Compound 1.

## Patentansprüche

1. Pharmazeutische Zusammensetzung zur Verwendung in einem Verfahren zur Behandlung von T2DM, umfassend die Verabreichung der pharmazeutischen Zusammensetzung an einen Menschen, der diese benötigt,
wobei die pharmazeutische Zusammensetzung in einer festen oralen Darreichungsform mit sofortiger Freisetzung vorliegt;
die pharmazeutische Zusammensetzung 2-[(4-{6-[(4-Cyano-2-fluorbenzyl)oxy]pyridin-2-yl}piperidin-1-yl)methyl]-1-[(2S)-oxetan-2-ylmethyl]-1H-Benzimidazol-6-carbonsäure (Verbindung 1) oder ein pharmazeutisch akzeptables Salz davon enthält;
die pharmazeutische Zusammensetzung zweimal täglich verabreicht wird; und
die pharmazeutische Zusammensetzung die Verbindung 1 oder ein pharmazeutisch akzeptables Salz davon in einer Menge, die 70 mg bis 120 mg der Verbindung 1 entspricht, enthält.

2. Pharmazeutische Zusammensetzung zur Verwendung in einem Verfahren zur Behandlung von T2DM, wie in Anspruch 1 beansprucht, wobei die pharmazeutische Zusammensetzung in einer Tablettendosierungsform mit sofortiger Freisetzung vorliegt.

3. Pharmazeutische Zusammensetzung zur Verwendung in einem Verfahren zur Behandlung von T2DM, wie in einem der Ansprüche 1 bis 2 beansprucht, wobei die Verbindung 1 oder ein pharmazeutisches Salz davon in der pharmazeutischen Zusammensetzung ein Tris-Salz der Verbindung 1 ist.

## Revendications

1. Composition pharmaceutique pour une utilisation dans un procédé de traitement du diabète sucré de type 2, T2DM, comprenant l'administration, à un humain en ayant besoin, de la composition pharmaceutique, dans laquelle :
la composition pharmaceutique se présente sous une forme posologique solide par voie orale à libération immédiate ;
la composition pharmaceutique comprend de l'acide 2-[(4-{6-[(4-cyano-2-fluorobenzyl)oxy]pyridin-2-yl}pipéridin-1-yl)méthyl]-1-[(2S)-oxétan-2-ylméthyl]-1H-benzimidazole-6-carboxylique (Composé 1) ou un sel pharmaceutiquement acceptable de celui-ci ;
la composition pharmaceutique est administrée deux fois par jour ; et
la composition pharmaceutique contient le Composé 1 ou un sel pharmaceutiquement acceptable de celui-ci en une quantité équivalente à 70 mg à 120 mg de Composé 1.

2. Composition pharmaceutique pour une utilisation dans un procédé de traitement du T2DM selon la revendication 1, dans laquelle la composition pharmaceutique se présente sous une forme posologique de comprimé à libération immédiate.

3. Composition pharmaceutique pour une utilisation dans un procédé de traitement du T2DM selon l'une quelconque des revendications 1 à 2, dans laquelle le Composé 1 ou un sel pharmaceutiquement acceptable de celui-ci dans la composition pharmaceutique est un sel tris du Composé 1.
